# EUROPEAN PATENT APPLICATION

(11) **EP 4 137 162 A1**
(43) Date of publication of application: **22.02.2023**
(21) Application number: 21789370.0
(22) Date of filing: 16.04.2021
(51) Int. Cl.: A61L 2/08, A61M 39/04, A61M 37/00

(54) **MEDICAL DEVICE AND MEDICAL APPARATUS**

(30) Priority: 17.04.2020 JP 2020073803
(71) Applicant: Furukawa Electric Co., Ltd., Tokyo 100-8322 (JP)
(72) Inventor: HIMURA Atsushi, Tokyo 100-8322 (JP); YAGI Takeshi, Tokyo 100-8322 (JP); NARA Kazutaka, Tokyo 100-8322 (JP); SUEMATSU Katsuki, Tokyo 100-8322 (JP); ARAI Tsunenori, Tokyo 100-8322 (JP)
(74) Representative: Winter, Brandl - Partnerschaft mbB
(86) International application number: PCT/JP2021/015778
(87) International publication number: WO 2021/210687

(57) **Abstract**

An exemplary embodiment of the present disclosure provides a medical instrument that makes it possible to reduce or prevent infection, and a medical device including the medical instrument. An implantable medical instrument for use within a body includes a container 12 that has an opening 12a and holds a medicinal solution, and a soft portion 13 that closes the opening 12a. The medical instrument further includes: a power receiver 15 that receives power transmitted externally; and a light emitter 16 that emits light by way of the power received by the power receiver 15. The light emitter 16 includes at least one of a first light emitter 16a that emits light having a center wavelength of 600 nm or more and 1100 nm or less, and a second light emitter 16b that emits light having a center wavelength of 400 nm or more and 480 nm or less.

## Description

### TECHNICAL FIELD

The present disclosure relates to an implantable medical instrument for use within the body of a subject, and a medical device including the medical instrument.

### BACKGROUND ART

A body-implantable medical device in which a body portion of a medical instrument is implanted into a body is used for charging a medicinal solution into the body (for example, refer to Japanese Unexamined Patent Application, Publication No. 2007-203070)
This medical instrument reduces the burden on patients who need frequent injections for charging a medicinal solution. Such a medical instrument has a soft portion through which an injection needle is inserted into the body portion. This soft portion is made of, for example, silicone rubber or other materials. With the medical instrument, a medicinal solution is injected into a medicinal solution container through the soft portion. The medicinal solution is transported through a catheter to blood vessels.

Patent Document 1: Japanese Unexamined Patent Application, Publication No. 2007-203070

### DISCLOSURE OF THE INVENTION

### Problems to be Solved by the Invention

As described above, after this medical instrument is implanted within the patient's body, it is necessary to puncture periodically with an injection needle for charging a medicinal solution. However, puncturing with an injection needle may cause bacteria to grow in the skin covering the medical instrument and in the interior of the medical instrument including the soft portion, thereby causing an infection. Therefore, it is desired to reduce or prevent infection.

An exemplary embodiment of the present disclosure provides a medical instrument that makes it possible to reduce or prevent an infection, and a medical device including the medical instrument.

### Means for Solving the Problems

An exemplary embodiment of the present invention provides an implantable medical instrument for use within a body, the medical instrument including a container that has an opening and holds a medicinal solution, and a soft portion that closes the opening, the medical instrument including: a power receiver that receives power transmitted externally; and a light emitter that emits light by way of the power received by the power receiver, in which the light emitter includes at least one of a first light emitter that emits light having a center wavelength of 600 nm or more and 1100 nm or less, and a second light emitter that emits light having a center wavelength of 400 nm or more and 480 nm or less.

Furthermore, in the above medical instrument, the light emitter is provided around the soft portion, and irradiates at least a top surface of the soft portion.

Furthermore, in the above medical instrument, the light emitter is disposed around a middle portion or a bottom portion of the container, and irradiates at least an interior of the container and the soft portion.

Furthermore, in the above medical instrument, the light emitter includes at least the first light emitter, and a radiant exposure of the light emitted from the first light emitter is in a range from 1.0 to 100.0 J/cm2 at an irradiation target site.

Furthermore, in the above medical instrument, the light emitter includes at least the first light emitter, and the first light emitter emits light having a center wavelength of 620 nm, 660 nm, 680 nm, 760 nm, or 820 nm.

Furthermore, in the above medical instrument, the light emitter includes at least the second light emitter, and a radiant exposure of the light emitted from the second light emitter is in a range from 32 to 125 J/cm2 at an irradiation target site.

An exemplary embodiment of the present invention provides a medical device including a medical instrument according to any one of the above medical instruments; and a power transmitter that transmits predetermined power, in which the medical instrument further includes a power receiver that receives power transmitted from the power transmitter.

### Effects of the Invention

According to an exemplary embodiment of the present disclosure, it is possible to reduce or prevent an infection.

### BRIEF DESCRIPTION OF THE DRAWINGS

FIG. 1 is a diagram schematically showing a schematic configuration of a medical device according to an exemplary embodiment of the present disclosure.
FIG. 2 is a diagram schematically showing a side cross-section of a medical instrument.
FIG. 3 is a diagram schematically showing a top surface of a medical instrument.
FIG. 4 is a diagram schematically showing a side cross-section of a medical instrument.
FIG. 5 is a diagram showing a configuration example of a power receiver and a light emitter.
FIG. 6 is a diagram schematically showing a side cross-section of a medical instrument according to a first arrangement example.
FIG. 7 is a diagram schematically showing a side cross-section of a medical instrument according to a second arrangement example.
FIG. 8 is a diagram schematically showing an external configuration of a container according to the second arrangement example.
FIG. 9 is a diagram schematically showing a side cross-section of a medical instrument according to a third arrangement example.
FIG. 10 is a diagram schematically showing a side cross-section of a medical instrument according to a fourth arrangement example.
FIG. 11 is a diagram schematically showing an external configuration of a container according to the fourth arrangement.
FIG. 12 is a diagram schematically showing a side cross-section of a medical instrument according to a fifth arrangement example.
FIG. 13 is a diagram schematically showing an external configuration of a container according to the fifth arrangement example.
FIG. 14 is a diagram schematically showing a side cross-section of a medical instrument according to a sixth arrangement example.
FIG. 15 is a diagram schematically showing a side cross-section of a medical instrument according to a seventh arrangement example.
FIG. 16 is a diagram schematically showing a side cross-section of a medical instrument according to an eighth arrangement example.
FIG. 17 is a diagram schematically showing a side cross-section of a medical instrument according to a ninth arrangement example.
FIG. 18 is a diagram schematically showing a side cross-section of the medical instrument according to the tenth arrangement example.
FIG. 19 is a diagram schematically showing a side cross-section of a medical instrument according to an eleventh arrangement example.
FIG. 20 is a diagram schematically showing a top surface of the medical instrument according to the eleventh arrangement example.
FIG. 21 is a diagram schematically showing a side cross-section of a medical instrument according to a twelfth arrangement example.
FIG. 22 is a diagram schematically showing a top surface of the medical instrument according to the twelfth arrangement example.
FIG. 23 is a diagram schematically showing a side cross-section of a medical instrument according to a thirteenth arrangement example.
FIG. 24 is a diagram schematically showing a top surface of the medical instrument according to the thirteenth arrangement example.
FIG. 25A is an image showing a status of methicillin-resistant Staphylococcus aureus (MRSA) colonies formed on a control agar-culture medium after 48 hours of incubation.
FIG. 25B is an image showing the status of methicillin-resistant Staphylococcus aureus (MRSA) colonies formed on an agar-culture medium after red-light irradiation at a radiant exposure of 60 [J/cm²] and after 48 hours of incubation.

### PREFERRED MODE FOR CARRYING OUT THE INVENTION

Hereinafter, exemplary embodiments for carrying out the present disclosure will be described in detail with reference to the accompanying drawings. It should be noted that the present disclosure is not limited to the following exemplary embodiments. In addition, the drawings referred to in the following description are merely schematic representations of the shape, size, and positional relationship to the extent that the content of the present disclosure can be understood. That is, the present disclosure is not limited to the shapes, sizes, and positional relationships illustrated in the drawings. Furthermore, in the following description, configurations and operations of an implantable medical instrument for use within a living body of a subject including a human and an animal, and a medical device including the medical instrument will be described in detail.

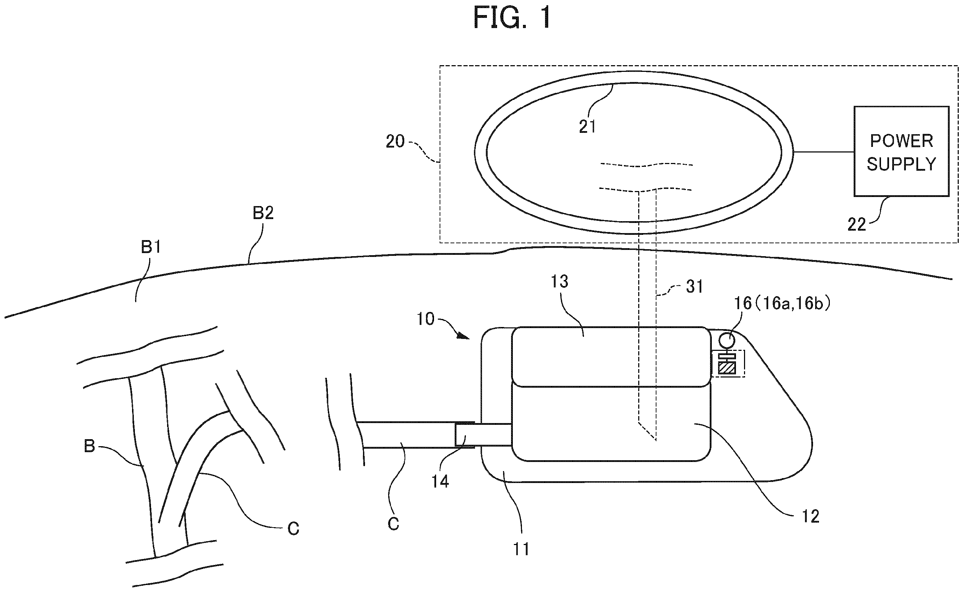

FIG. 1 schematically shows a state when a medical instrument 10 is implanted into the body of a patient, and FIG. 1 is a diagram schematically showing a configuration of a medical device 1. The medical device 1 includes the medical instrument 10 and a power transmitter 20. The medical instrument 10 is, for example, referred to as a subcutaneous implantable port (CV port), and is an implantable instrument for use which includes a container having an opening closed by a soft portion and holding a medicinal solution therein. FIG. 1 schematically shows a state in which the medical instrument 10 is implanted in a patient's body B1.

### (Medical Instrument)

The configuration and operation of the medical instrument 10 will be described below. The medical instrument 10 includes a soft portion 13 through which an injection needle 31 is inserted, and a container 12 that holds a medicinal solution injected by the injection needle 31. In this exemplary embodiment, a surface of the soft portion 13 through which the injection needle 31 is inserted is referred to as a top surface of the medical instrument 10, a surface opposing the top surface is referred to as a bottom surface of the medical instrument 10, and a surface connecting the top surface and the bottom surface is referred to as a side surface of the medical instrument 10.

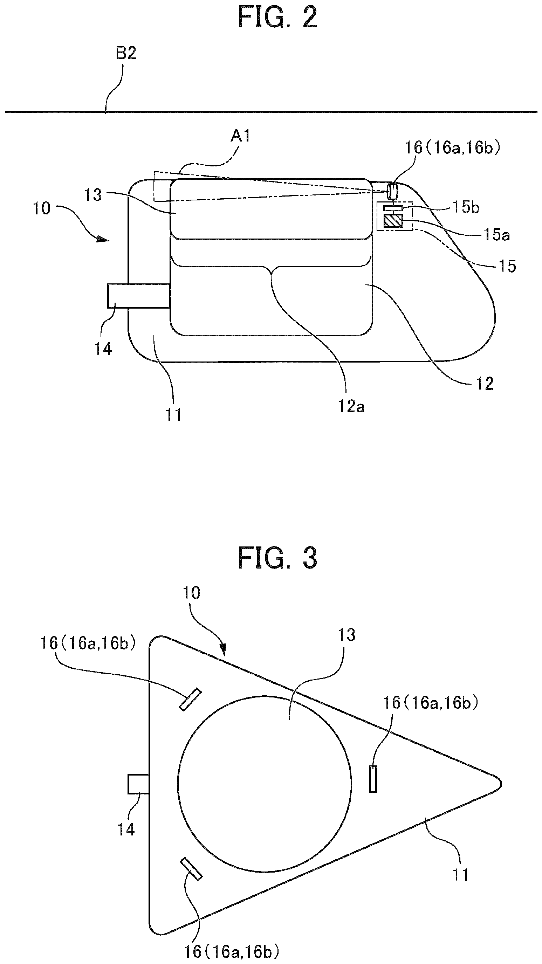

FIG. 2 is a diagram schematically showing a side cross-section of the medical instrument 10. FIG. 3 is a diagram schematically showing a top surface of the medical instrument 10. As shown in FIG. 2, the medical instrument 10 mainly includes a main body 11, the container 12 having an opening 12a and holding a medicinal solution, and the soft portion 13 that closes the opening 12a of the container 12. Furthermore, the medical instrument 10 includes an injector (catheter port) 14 to which a catheter C is connected and which injects a solution contained in the container 12 into a blood vessel (vein) B through the catheter C.

The main body 11 is, for example, a housing made of epoxy resin or other materials. The container 12 is a tank that temporarily stores a medicinal solution. The container 12 includes the opening 12a in the vicinity of the outside of the living body. The opening 12a has a circular or substantially circular shape. FIG. 2 shows that the opening 12a is closed by the soft portion 13. The container 12 may be made entirely of metal, may be made entirely of resin, or may be made of metal only at the bottom portion and may be made of resin at the other portions than the bottom portion.

The soft portion 13 is referred to as a septum. The soft portion 13 closes the opening 12a of the container 12. The soft portion 13 is, for example, a soft lid body (silicone diaphragm) made of silicone rubber, and is exposed from the main body 11 as well. Furthermore, the soft portion 13 has a columnar or substantially columnar shape. However, it may have a shape other than a columnar shape. The soft portion 13 is a portion through which the injection needle 31 for the injection (infusion) of a medicinal solution can be inserted from a body surface of a subject after the main body 11 is implanted within the living body B1. It should be noted that FIG. 1 schematically shows the external shape of the medical instrument 10. Therefore, various shapes may be available to the main body 11, the container 12, and other components. The injection needle 31 is, for example, a Huber needle. After being inserted into the soft portion 13, a medicinal solution or other liquid is injected into the container 12 from the tip of the injection needle 31.

Furthermore, the medical instrument 10 includes a power receiver 15 and light emitters 16. The power receiver 15 receives power transmitted from the outside (a power transmitter 20). The light emitters 16 are for example, LEDs (light emitting diode), and each emits light by the power received by the power receiver 15. Furthermore, the light emitters 16 each include at least one of a first light emitter 16a that emits light having a center wavelength of 600 nm or more and 1100 nm or less, and a second light emitter 16b that emits light having a center wavelength of 400 nm or more and 480 nm or less. That is, the light emitter 16 may include only the first light emitter 16a, the light emitter 16 may include only the second light emitter 16b, or the light emitter 16 may include both the first light emitter 16a and the second light emitter 16b. In the present exemplary embodiment, when expressed as "light emitter 16", the "light emitter 16" indicates the first light emitter 16a or the second light emitter 16b. The range on the long wavelength side (600 nm or more and 1100 nm or less) and the range on the short wavelength side (400 nm or more and 480 nm or less) described above were each specified by the inventors and other by undertaking extensive effort.

The light emitters 16 are disposed around the soft portion 13, and irradiate at least the top surface of the soft portion 13. For example, the light emitters 16 are disposed in an annular or substantially annular manner at predetermined intervals around the soft portion 13. More specifically, when three light emitters 16 are used, each of the light emitters 16 is disposed in an annular or substantially annular manner around the soft portion 13 at intervals of about 120 degrees, as shown in FIG. 3. The light emitter 16 may be sloped by a predetermined angle toward the center of the soft portion 13 so as to irradiate the top surface of the soft portion 13. It should be noted that the number of light emitters 16 and the position and angle to be arranged can be appropriately changed.

With such a configuration, the medical instrument 10 can irradiate at least the top surface of the soft portion 13 by arranging the light emitters 16 around the soft portion 13, such that infection caused on the top surface of the soft portion 13 can be reduced or prevented, as described below.

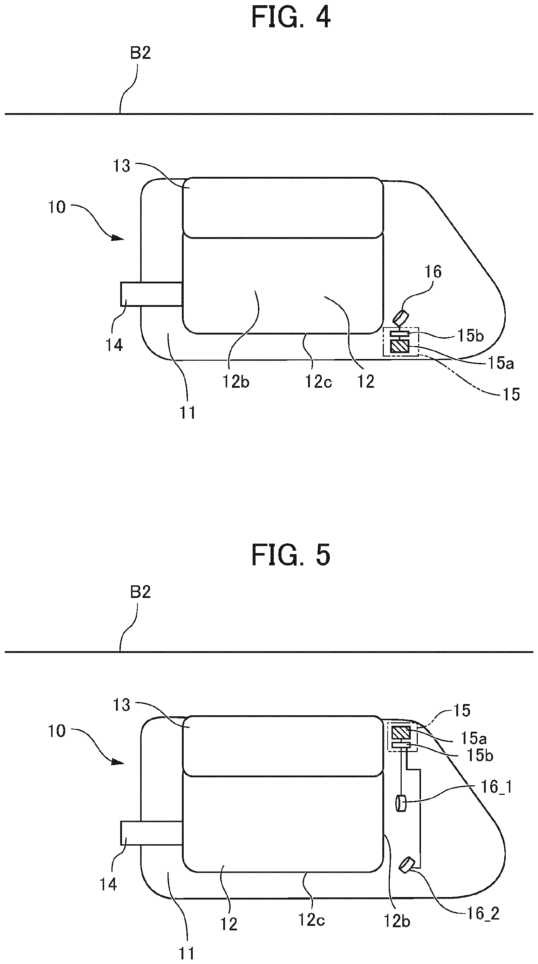

Furthermore, the light emitter 16 may be disposed around a middle portion 12b or a bottom portion 12c of the container 12 to irradiate at least the interior of the container 12 and the soft portion 13. FIG. 4 is a diagram schematically showing a side cross-section of the medical instrument 10. More specifically, FIG. 4 schematically shows an example in which the light emitter 16 is disposed around the bottom portion 12c of the container 12.

With such a configuration, the medical instrument 10 can irradiate at least the interior of the container 12 and the soft portion 13 by disposing the light emitter 16 around the middle portion 12b or around the bottom portion 12c of the container 12, such that infection caused in the interior of the container 12 and the soft portion 13 can be reduced or prevented.

In FIG. 2, although an irradiation site (range) A1 by the light emitter 16 is shown schematically so that it may target only a portion beneath the skin (the surface of the soft portion 13), the irradiation site (range) by the light emitter 16 is not limited to this region. Although a detailed configuration will be described later, it is possible to irradiate various sites by changing the position and angle where the light emitter 16 is disposed, and the number of the light emitters 16. The various sites may include not only the portion beneath the skin (the surface of the soft portion 13), but also the following sites, for example.
1. All layers of the skin (including epidermis and dermis) only
2. All layers of the skin and portions beneath the skin (the surface of the soft portion 13)
3. All layers of the skin and all layers of the soft portion 13
4. All layers of the skin, all layers of the soft portion 13, and all layers of the container 12 (the entire container 12)
5. All layers of the skin, all layers of the soft portion 13, all layers of the container 12, and entire injector 14

In addition, the range of radiant exposure (radiation dose) E1 of the light emitted from the first light emitter 16a can be set as, for example, 1.0 to 100.0 [J/cm²] at the irradiation target site from the viewpoint of influences on the human body and working efficiencies. Here, the radiant exposure will be described. The radiant exposure E is calculated by an irradiance I of the illumination (irradiation) and the irradiation time t as shown in expression (1). The irradiance refers to the radiant flux incident per unit area. E[J/cm²] = I[W/cm²] × t[s] ...(1)

For example, when the irradiance I is "4.5 × 10⁻³[W/cm²]" and the exposure time t is about "1100[s]", then the radiant exposure E1 is "4.95 [J/cm²]". In addition, when the irradiance I is "10 × 10⁻³[W/cm²]" and the exposure time t is about "550[s]", the radiant exposure E1 is "5.5 [J/cm²]". It should be noted that the range of the irradiance I can be set as 4 to 500 [mW per cm²]. The irradiance I can be measured, for example, by using a spectroradiometer (JIS C 1609-1: 2006 general class AA illuminometer) .

Furthermore, the radiant exposure E1 may range from 1.0 to 100.0 [J/cm²]. In a living body environment in which neutrophils, which are blood components, are present, the radiant exposure E1 ranges preferably 1.0 to 20.0 [J/cm²], and more preferably 2.0 to 8.8 [J/cm²]. In a living body environment in which neutrophils are not present, for example in the stratum corneum of the skin, the radiant exposure E1 ranges preferably 1 to 100 [J/cm²], more preferably 1 to 60 [J/cm²], and even more preferably 10 to 60 [J/cm²].

Furthermore, the first light emitter 16a emits light having a center wavelength of 620 [nm], 660 [nm], 680 [nm], 760 [nm], or 820 [nm]. It should be noted that, when the center wavelength falls in the range of X±10 [nm], the center wavelength can be regarded as X [nm]. Furthermore, the center wavelengths described above are merely examples, and are not limited to these wavelengths.

Furthermore, the radiant exposure E2 of the light emitted from the second light emitter 16b may range from 32 to 125 [J/cm²] at the irradiation target site. More specifically, for example, the radiant exposure E2 is 125 [J/cm²], preferably 62 [J/cm²], and more preferably 32 [J/cm²].

For example, when the irradiance I is "8.74×10⁻³[W/cm²]" and the irradiation time t is about "3600 [s]", the radiant exposure E2 is "31.46 [J/cm²]". In addition, when the irradiance I is "8.6×10⁻³[W/cm²]" and the irradiation time t is about 7200 [s], the radiant exposure E2 is "61.92 [J/ cm²]". It should be noted that the range of irradiance I may be 7 to 12 [mW/cm²].

Here, the effect of the light emitted from the first light emitter 16a, i.e., reduction or prevention of an infection, will be described. The living body effect produced when light in the red region or the near infrared region is irradiated with the irradiance and radiant exposure to such an extent that no thermal action occurs in the living body is called Photobiomodulation (PBM). In PBM, the irradiation light is considered to be absorbed by an enzyme in the respiratory metabolism mechanism of the cell, and various effects are expressed depending on the situations of the cell and the living tissue.

In addition, various effects such as healing of infected wounds and anti-inflammatory effect have been reported by performing PBM. These are known as typical PBM effects. In this disclosure, these effects are collectively referred to as reduction or prevention of an infection, or infection control. In addition, the following paper is referred to for the PBM irradiation conditions and mechanism. T Walski et al., "The effect of red-to-near-infrared (R/NIR) irradiation on inflammatory processes", Int J Rad Biol, Vol. 95, No. 9, p.1326-1336, 2019.

Next, the effect of the light emitted from the second light emitter 16b, i.e., reduction or prevention of an infection, will be described. The effect of irradiation with light (blue light) on the disinfection of pathogenic microorganisms (e.g., Staphylococcus aureus) has been reported in the following paper. Yucheng Wang, Ying Wang, Yuguang Wang, Clinton K. Murray, Michael R. Hamblin, David C. Hooper, Tianhong Dai, "Antimicrobial blue light inactivation of pathogenic microbes", Drug Resistance Updates Vol. 33-35, P. 1-22 (2017)

Furthermore, the effective depth (distance) in the biological tissue is considered to be less than 1 (mm). Therefore, the second light emitter 16b is not suitable for the sterilization of biological tissues (other than the irradiated surface), and thus, it is considered to be used for the sterilization of, for example, the surface of the container 12 and the surface of the injector 14, or the sterilization of a transparent portion.

### (Power Transmitter)

The configuration and operation of the power transmitter 20 will be described. The power transmitter 20 transmits predetermined power. The power transmitter 20 includes a second coil 21 and a power supply 22. The second coil 21 has an annular or substantially annular shape of a predetermined diameter (for example, approximately the outer diameter of the soft portion to be described later), and generates a magnetic flux corresponding to the power supplied from the power supply 22. The power receiver 15 of the medical instrument 10 receives power transmitted from the power transmitter 20.

### (Power Receiver)

As shown in FIG. 2, the power receiver 15 includes a first coil 15a and a power receiving circuit 15b. The first coil 15a receives power transmitted from the power transmitter 20. The power receiving circuit 15b uses the power generated in the first coil 15a (induced electromotive force), to cause the light emitter 16 to emit light.

Although an example is shown in which one light emitter 16 is provided, the number of light emitters 16 is not limited to one, and may be two or more.

Furthermore, FIG. 2 shows an example in which the light emitter 16 and the power receiver 15 are provided as a set. However, the light emitter 16 and the power receiver 15 may not be provided as a set. FIG. 5 is a diagram showing an example in which one power receiver 15 and two light emitters 16_1 and 16_2 are provided. The power receiver 15 is disposed in the vicinity of the soft portion 13. The vicinity of the soft portion 13 refers to the interior or the surface of the main body 11 in the vicinity where the soft portion 13 is located. In this manner, since the power receiver 15 is disposed in the vicinity of the soft portion 13, and the power receiver 15 is located at a location in the vicinity of the skin B2 when the medical instrument 10 is implanted in the body, the power receiver 15 can receive the power transmitted from the power transmitter 20 with high sensitivity.

In addition, the light emitter 16_1 is disposed around the middle portion 12b of the container 12. The light emitter 16_2 is disposed around the bottom portion 12c of the container 12. It should be noted that the light emitters 16_1 and 16_2 may be provided at locations other than those described above.

With such a configuration, since the medical instrument 10 causes the light emitter 16_1 and the light emitter 16_2 to emit light by the power received by the power receiver 15, respectively, it is possible to irradiate various regions (for example, all layers of the soft portion 13 and all layers of the container 12, etc.), such that it is possible to reduce or prevent an infection on various regions. It should be noted that the power receiver 15 may cause the light emitter 16_1 and the light emitter 16_2 to emit light at the same time, or alternatively, may cause them to emit light with a time difference. Furthermore, the light emitter 16_1 and the light emitter 16_2 may have the same center wavelength or different center wavelengths. For example, the light emitter 16_1 may be the first light emitter 16a, and the light emitter 16_2 may be the second light emitter 16b. Furthermore, the light emitter 16_1 may be the second light emitter 16b, and the light emitter 16_2 may be the first light emitter 16a.

In addition, the medical instrument 10 can reduce or prevent infection by the anti-inflammatory effect on the irradiated portion by causing the light emitter 16 to emit light by the power transmitted from the power transmitter 20 at a predetermined time period (for example, before injecting the medicinal solution into the container 12 by the injection needle 31) and irradiating a predetermined location (for example, the top surface of the soft portion 13) with the light.

### (Principle of Anti-inflammatory Effect)

Here, the principle of the anti-inflammatory effect will be explained. It has been reported that PBM using light of a predetermined wavelength (for example, 660 [nm]) in a living body has an effect of reducing or preventing the growth of Staphylococcus aureus, which is the most common bacterium related to infection. It is thought to be effective in reducing or preventing the respiratory metabolism of Staphylococcus aureus and in enhancing the phagocytosis and bactericidal action of neutrophils that eliminate bacteria in the organism.

Neutrophils generally phagocytize bacteria, produce ROS, H₂O₂, and the like by NADPH oxidase, and sterilize bacteria. Furthermore, neutrophils generate hypochlorous acid from the produced H₂O₂ and hydrochloric acid ions and sterilize bacteria.

[Mechanism 1] PBM using light at a wavelength of 660 [nm] increases oxide emission from neutrophils. Thus, the bactericidal effect of neutrophils is enhanced.

[Mechanism 2] PBM affects the respiratory metabolic pathway of Staphylococcus aureus and reduces its activity. As a result, Staphylococcus aureus becomes vulnerable to oxidative stress. Therefore, the effects of ROS and H₂O₂ produced by the above-mentioned neutrophils are enhanced.

Inactivation (alternatively) of two final enzymes in the respiratory pathway of Staphylococcus aureus results in a reduction in oxidative resistance similar to a result from PBM, and further addition of PBM does not further enhance the effect.

From the above, it is considered that irradiation with light having a wavelength of 660 nm inhibits respiratory metabolism of Staphylococcus aureus and results in oxidation resistance.

Here, it has been reported in the following paper that the anti-inflammatory effect was confirmed when PBM was performed using light having a wavelength of 685 [nm] on the wound of a rat infected with bacteria. Acta Cirúrgica Brasileira, Vol. 31, No. 8, pp. 489-504 (2016).

In addition, it has been reported in the following paper that PBM using light having a wavelength of 660 [nm] was performed increased oxide emission from neutrophils and enhanced phagocytic ability. Journal of Biomedical Optics, Vol. 9, No. 11-12, pp. 1180-1188 (2016).

In addition, it has been reported in the following paper that PBM using light having a wavelength of 660 [nm] confirmed the effect of reducing the oxidation resistance of Staphylococcus aureus. Oxidative Medicine and Cellular Longevity, Vol. 2018, ID 6510519 (11 pages) (2018).

As described above, by PBM with red light, it is considered that neutrophils (blood components) cause an anti-inflammatory effect (sterilization). In addition, it has been reported that PBM inhibits the aerobic metabolism of Staphylococcus aureus in the absence of neutrophils, but this effect inhibits the proliferation (for example, refer to the following paper). Lasers in Medical Science, Vol. 31, No. 3, pp. 549-556 (2016).

In addition, it has also been reported that the active sterilization in the environment in the absence of neutrophils is effective by irradiating with blue light (for example, light at a wavelength of 465 [nm]) (for example, refer to the following papers). Lasers in Medical Science, Vol. 34, No. 9, pp. 1799-1805 (2019). Veterinary Dermatology, Vol. 28, No. 9, pp. 463-e106 (2017).

### (Marker Function)

Furthermore, as described above, in the medical instrument 10, the light emitter 16 implanted in the body emits light in accordance with the proximity of the power transmitter 20 (the second coil 21). This light emission can also be visually recognized from outside the body. Furthermore, the top surface of the soft portion 13 corresponds to the location where the light is emitted.

Therefore, when injecting the medicinal solution into the container 12 by the injection needle 31, it is possible to insert the injection needle 31 thereinto with this light emitter as a mark (marker function) by causing the light emitter 16 to emit light by using the power transmitter 20. Thus, the position of the soft portion 13 of the medical instrument 10 is confirmed by way of the light of the light emitter 16, so that the injection needle 31 can be reliably inserted into the soft portion 13.

### (First Arrangement Example of Light Emitter)

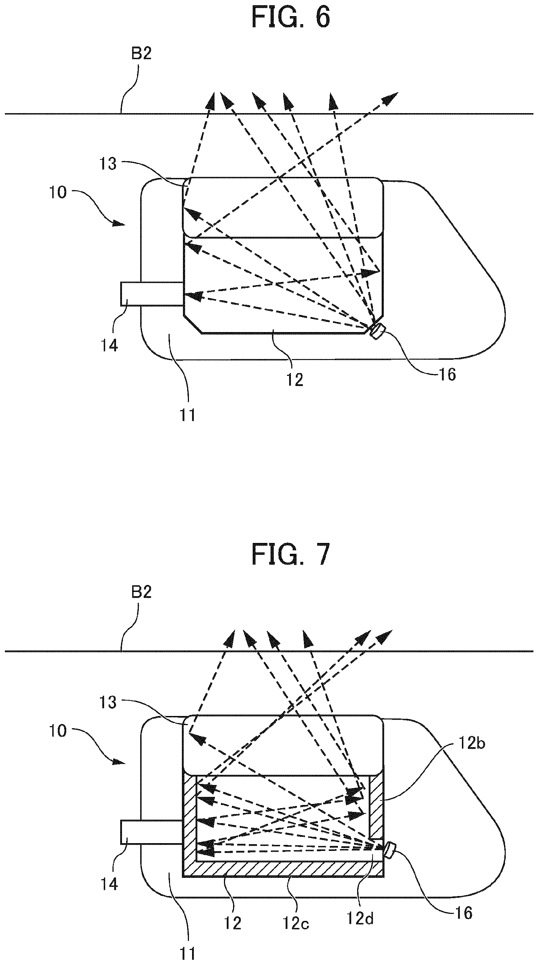

Here, a first arrangement example of the light emitter 16 will be described. FIG. 6 is a diagram schematically showing a side cross-section of the medical instrument 10 according to the first arrangement example. It should be noted that, for convenience of explanation, FIG. 6 schematically shows the light (rays) emitted from the light emitter 16. In an actual situation, the light emitted from the light emitter 16 is emitted with a predetermined spread (total radiant flux). The radiant flux is a unit of light that represents the radiant energy passing through a surface per unit of time. The total radiant flux refers to the total amount of radiant energy of light emitted in all directions from the light emitter 16 which is a light source.

The container 12 is made entirely of resin. The light emitters 16 are disposed around the edge of the bottom portion 12c of the container 12. More specifically, three light emitters 16 are arranged at intervals of 120 degrees around the edge of the bottom portion 12c of the container 12. The mounting position and angle of the light emitter 16 is determined so that the light emitted from the light emitter 16 is scattered in the entire container 12, and incident from the lower surface of the soft portion 13 so as to pass upward through the soft portion 13. For example, it is considered that the light emitted from the light emitter 16 is scattered to the entire container 12 by providing the inner wall of the container 12 having an appropriate roughness. It should be noted that the number of the light emitters 16 is not limited to three.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, and all layers of the skin. Furthermore, according to the first arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12.

### (Second Arrangement Example of Light Emitter)

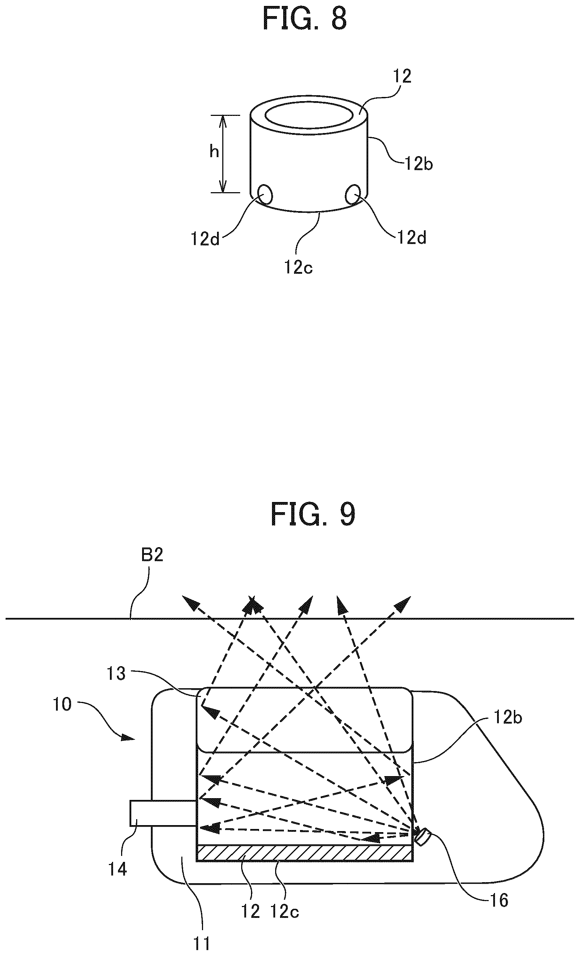

FIG. 7 is a diagram schematically showing a side cross-section of the medical instrument according to the second arrangement example. FIG. 8 is a diagram schematically showing an external configuration of the container 12. The container 12 is made entirely of metal. In the container 12, as shown in FIG. 7, three holes 12d are provided at intervals of about 120 degrees around the bottom portion 12c. It should be noted that the number of the holes 12d is not limited to three. Furthermore, the hole 12d may be provided around the middle portion 12b. The light emitter 16 is disposed for each hole 12d. In the example shown in FIG. 8, when the dimension in the height direction of the container 12 (i.e., the height from the bottom portion 12c to the upper end of the side surface of the container 12) is defined as h, the height from the hole 12d to the bottom portion 12c is smaller than h/3.

The light emitted from the light emitter 16 is reflected inside the metal container 12, and reaches all layers of the soft portion 13 and all layers of the skin from the lower surface of the soft portion 13.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, all layers of the skin, and other sites. Furthermore, according to the second arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12. Furthermore, in the second arrangement example, since the container 12 is made entirely of metal, light does not leak from the side surface of the container 12, and the reflection toward the soft portion 13 increases. Thus, since the intensity of the light released from the soft portion 13 becomes strong, it is possible to further improve the anti-inflammatory effect at the puncture position, and it is possible to more accurately grasp the position of the soft portion 13 from the outside on the top surface side of the soft portion 13. The hole 12d may be provided on the bottom surface of the container 12. In this configuration, the light emitter 16 emits light upward from the position (bottom surface) of the hole 12d of the container 12. In addition, the container 12 may be made of metal only on its side surface, and may be made of resin on its bottom surface.

### (Third Arrangement Example of Light Emitter)

FIG. 9 is a diagram schematically showing a side cross-section of the medical instrument 10 according to the third arrangement example. In the container 12, the bottom portion 12c is made of metal, and the middle portion 12b other than the bottom portion 12c is made of resin. The light emitter 16 is disposed around the bottom portion 12c of the container 12. More specifically, three light emitters 16 are disposed at intervals of about 120 degrees around the bottom portion 12c of the container 12. The mounting position and angle of the light emitter 16 is determined so that the light emitted from the light emitter 16 is scattered in the entire container 12, and incident from the lower surface of the soft portion 13 so as to reach all layers of the soft portion 13, all layers of the skin, and other sites. For example, it is considered that the light emitted from the light emitter 16 is scattered to the entire container 12 by providing the inner wall of the container 12 having an appropriate roughness. The number of the light emitters 16 is not limited to three.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, all layers of the skin, and other sites. Furthermore, according to the third arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12.

### (Fourth Arrangement Example Light Emitter)

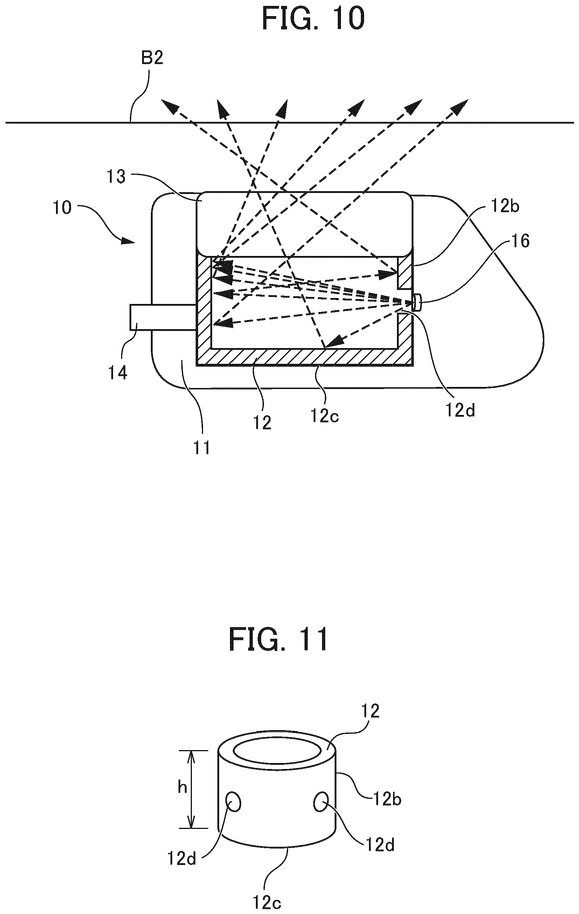

FIG. 10 is a diagram schematically showing a side cross-section of the medical instrument 10 according to the fourth arrangement example. FIG. 11 is a diagram schematically showing the external configuration of the container 12 according to the fourth arrangement. The container 12 is made entirely of metal. Furthermore, in the fourth arrangement example, the position where the holes 12d are provided is different from that in the second arrangement example. More specifically, as shown in FIG. 11, the container 12 has three holes 12d provided at intervals of about 120 degrees around the center of the middle portion 12b. It should be noted that the number of the holes 12d is not limited to three. The light emitter 16 is disposed for each hole 12d. In the example of FIG. 11, when the dimension in the height direction of the container 12 is defined as h, the height from the hole 12d to the bottom portion 12c is h/3 or more, and is smaller than 2h/3.

The light emitted from the light emitter 16 is reflected inside the metal container 12, and reaches all layers of the soft portion 13 and all layers of the skin from the lower surface of the soft portion 13.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, all layers of the skin, and other sites. Furthermore, according to the fourth arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12. Furthermore, in the fourth arrangement example, since the container 12 is made entirely of metal, light does not leak from the side surface of the container 12, and the reflection toward the soft portion 13 increases. Thus, since the intensity of the light released from the soft portion 13 becomes strong, it is possible to further improve the anti-inflammatory effect at the puncture position, and it is possible to more accurately grasp the position of the soft portion 13 from the outside on the top surface side of the soft portion 13. In addition, the container 12 may be made of metal only on its side surface, and may be made of resin on its bottom surface.

### (Fifth Arrangement Example of Light Emitter)

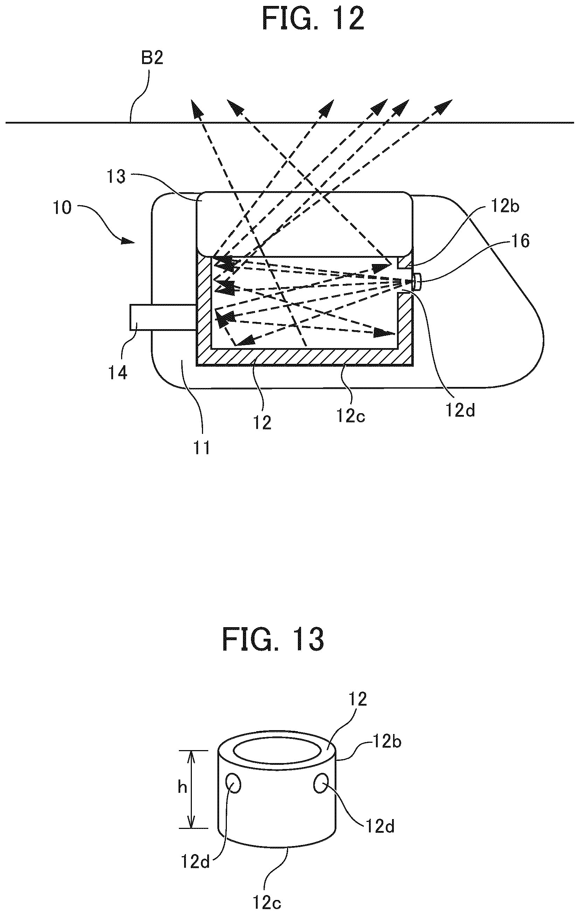

FIG. 12 is a diagram schematically showing a side cross-section of the medical instrument 10 according to the fifth arrangement example. FIG. 13 is a diagram schematically showing an external configuration of the container 12 according to the fifth arrangement example. The container 12 is made entirely of metal. In the fifth arrangement example, the position where the holes 12d are provided is different from that of the second arrangement example and the fourth arrangement example. More specifically, as shown in FIG. 13, the container 12 has three holes 12d provided at intervals of about 120 degrees around the vicinity of the upper portion of the middle portion 12b. The number of the holes 12d is not limited to three. The light emitter 16 is disposed at each hole 12d. In addition, it is considered that the direction (slope) of the light-emitting surface of the light emitter 16 when placing the light emitter 16 in each hole 12d is set as, for example, the downward direction (the direction of the bottom portion 12c of the container 12) and the horizontal direction. When the direction of the light-emitting surface of the light emitter 16 is placed in the horizontal direction, there is an advantage in that the degree of irradiation of light to the soft portion 13 is improved. In the example shown in FIG. 13, when the dimension in the vertical direction of the container 12 is defined as h, the height from the hole 12d to the bottom portion 12c is 2h/3 or more and is smaller than h.

The light emitted from the light emitter 16 is reflected inside the metal container 12, and reaches all layers of the soft portion 13 and all layers of the skin from the lower surface of the soft portion 13.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, all layers of the skin, and other sites. Furthermore, according to the fifth arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12. Furthermore, in the fifth arrangement example, since the container 12 is made entirely of metal, light does not leak from the side surface of the container 12, and the reflection toward the soft portion 13 increases. Thus, since the intensity of the light released from the soft portion 13 becomes strong, it is possible to further improve the anti-inflammatory effect at the puncture position, and it is possible to more accurately grasp the position of the soft portion 13 from the outside on the top surface side of the soft portion 13. In addition, the container 12 may be made of metal only on its side surface, and may be made of resin on its bottom surface.

### (Sixth Arrangement Example of Light Emitter)

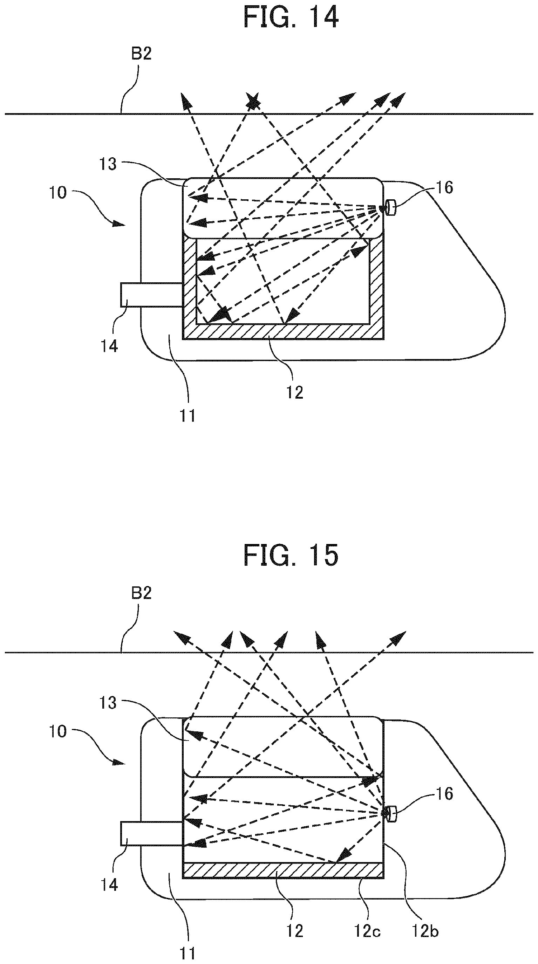

FIG. 14 is a diagram schematically showing a side cross-section of the medical instrument 10 according to the sixth arrangement example. The container 12 is made entirely of metal. In the fifth arrangement example, the hole 12d is not provided in the container 12. Three light emitters 16 are arranged at intervals of about 120 degrees around the soft portion 13. The number of the light emitters 16 is not limited to three.

The light emitted from the light emitter 16 is reflected inside the metal container 12, and reaches all layers of the soft portion 13 and all layers of the skin from the lower surface of the soft portion 13.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, all layers of the skin, and other sites. In particular, according to the sixth arrangement example, in the medical instrument 10, since it is possible to improve the degree of irradiation of light to the soft portion 13, it is possible to improve the reduction of or prevent infection in all layers of the soft portion 13. Furthermore, according to the sixth arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12. Further, in the sixth arrangement example, since the light emitter 16 is arranged to irradiate the soft portion 13 around the soft portion 13, the light is efficiently diffused by the soft portion 13, so that the anti-inflammatory effect can be further improved and the position of the soft portion 13 can be grasped more accurately from the outside on the top surface side of the soft portion 13.

### (Seventh Arrangement Example of Light Emitter)

FIG. 15 is a diagram schematically showing a side cross-section of the medical instrument 10 according to the seventh arrangement example. In the container 12, the bottom portion 12c is made of metal, and the middle portion 12b other than the bottom portion 12c is made of resin. In the seventh arrangement example, the position where the light emitters 16 are arranged is different from that in the third arrangement example. The light emitters 16 are disposed around the vicinity of the middle of the middle portion 12b of the container 12. More specifically, three light emitters 16 are arranged at intervals of 120 degrees around the vicinity of the middle of the middle portion 12b of the container 12. The mounting position and angle of the light emitter 16 is determined so that the light emitted from the light emitter 16 is scattered in the entire container 12, and incident from the lower surface of the soft portion 13 so as to reach all layers of the soft portion 13, all layers of the skin, and other sites. In addition, it is considered that the light emitted from the light emitter 16 is scattered to the entire container 12 by providing the inner wall of the container 12 having an appropriate roughness. The number of the light emitters 16 is not limited to three.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, all layers of the skin, and other sites. Furthermore, according to the seventh arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12.

### (Example of Eighth Arrangement of Light Emitter)

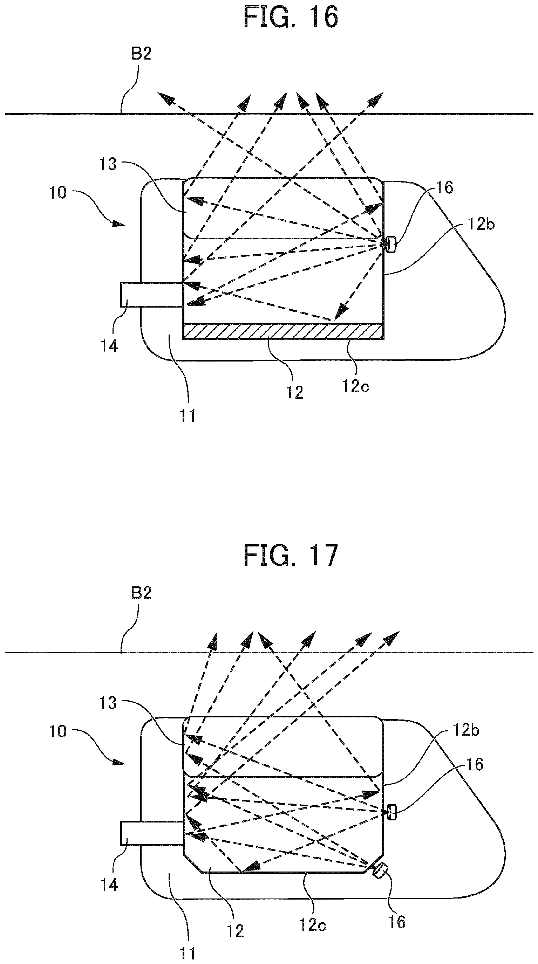

FIG. 16 is a diagram schematically showing a side cross-section of the medical instrument 10 according to the eighth arrangement example. In the container 12, the bottom portion 12c is made of metal, and the middle portion 12b other than the bottom portion 12c is made of resin. In the eighth arrangement example, the position where the light emitters 16 are arranged is different from that of the third arrangement example and the seventh embodiment. The light emitters 16 are disposed around the vicinity of the upper portion of the middle portion 12b of the container 12. More specifically, three light emitters 16 are arranged at intervals of 120 degrees around the vicinity of the upper portion of the middle portion 12b of the container 12. The mounting position and angle of the light emitter 16 are determined so that the light emitted from the light emitter 16 is scattered in the entire container 12, and incident from the lower surface of the soft portion 13 so as to reach all layers of the soft portion 13, all layers of the skin, and other sites. In addition, it is considered that the light emitted from the light emitter 16 is scattered to the entire container 12 by providing the inner wall of the container 12 having an appropriate roughness. The number of the light emitters 16 is not limited to three.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, all layers of the skin, and other sites. Furthermore, according to the eighth arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12.

### (Ninth Arrangement Example of Light Emitter)

FIG. 17 is a diagram schematically showing a side cross-section of the medical instrument 10 according to the ninth arrangement example. The container 12 is made entirely of resin. In the ninth arrangement example, the position where the light emitters 16 are arranged and the number of the light emitters 16 are different from those in the first arrangement example. The light emitters 16 are disposed around the edge of the bottom portion 12c of the container 12 and around the middle portion 12b. More specifically, three light emitters 16 are arranged at intervals of 120 degrees around the edge of the middle portion 12b of the container 12. In addition, three light emitters 16 are arranged at intervals of 120 degrees around the middle portion 12b of the container 12.

The mounting position and angle of the light emitter 16 is determined so that the light emitted from the light emitter 16 is scattered in the entire container 12, and incident from the lower surface of the soft portion 13 so as to reach all layers of the soft portion 13, all layers of the skin, and other sites. For example, it is considered that the light emitted from the light emitter 16 is scattered to the entire container 12 by providing the inner wall of the container 12 having an appropriate roughness. The number of the light emitters 16 is not limited to six.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, and all layers of the skin.

It should be noted that, in the ninth arrangement example, the entire container 12 is made of resin, but the present invention is not limited to this configuration, and the container 12 may be made entirely of metal, or the bottom portion may be made of metal (portions other than the bottom portion may be made of resin).

Furthermore, according to the ninth arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12.

### (Tenth Arrangement Example of Light Emitter)

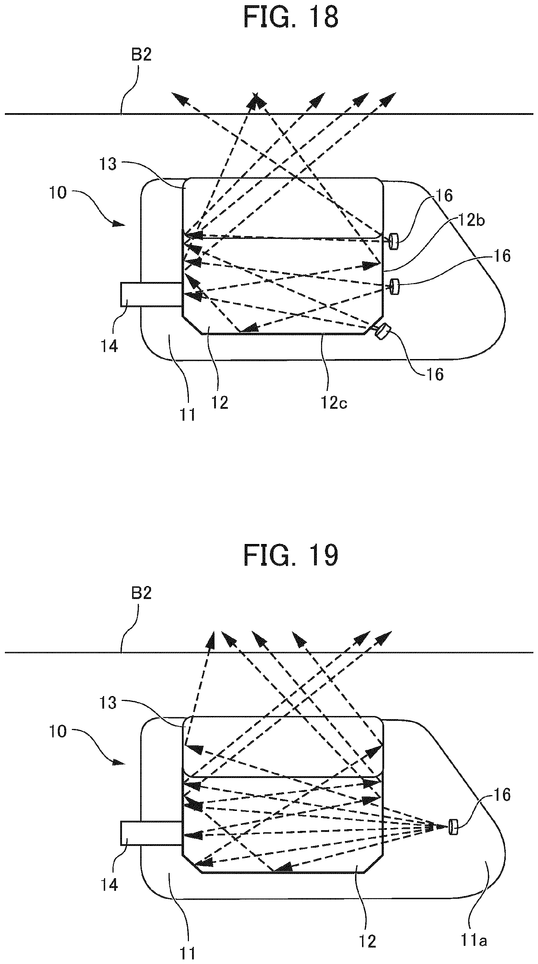

FIG. 18 is a diagram schematically showing a side cross-section of the medical instrument 10 according to the tenth arrangement example. The container 12 is made entirely of resin. In the tenth arrangement example, the position where the light emitters 16 are arranged and the number of the light emitters 16 are different from those of the first arrangement example and the ninth arrangement example. The light emitters 16 are disposed around the edge of the bottom portion 12c of the container 12, around the vicinity of the middle portion of the middle portion 12b, and around the vicinity of the upper portion of the middle portion 12b. More specifically, three light emitters 16 are arranged at intervals of 120 degrees around the edge of the middle portion 12b of the container 12. In addition, three light emitters 16 are arranged at intervals of 120 degrees around the vicinity of the center of the middle portion 12b of the container 12. In addition, three light emitters 16 are arranged at intervals of 120 degrees around the vicinity of the upper portion of the middle portion 12b of the container 12.

The mounting positions and angles of the respective light emitters 16 are determined so that the light emitted from the light emitter 16 is scattered in the entire container 12, and incident from the lower surface of the soft portion 13 so as to reach all layers of the soft portion 13. For example, it is considered that the light emitted from the respective light emitters 16 is scattered to the entire container 12 by providing the inner wall of the container 12 having an appropriate roughness. The number of the light emitters 16 is not limited to nine.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, and all layers of the skin.

It should be noted that, in the tenth arrangement example, the entire container 12 is made of resin, but the present invention is not limited to this configuration, and the container 12 may be made entirely of metal, or the bottom portion may be made of metal (portions other than the bottom portion may be made of resin).

Furthermore, according to the tenth arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12.

### (Eleventh Arrangement Example of Light Emitter)

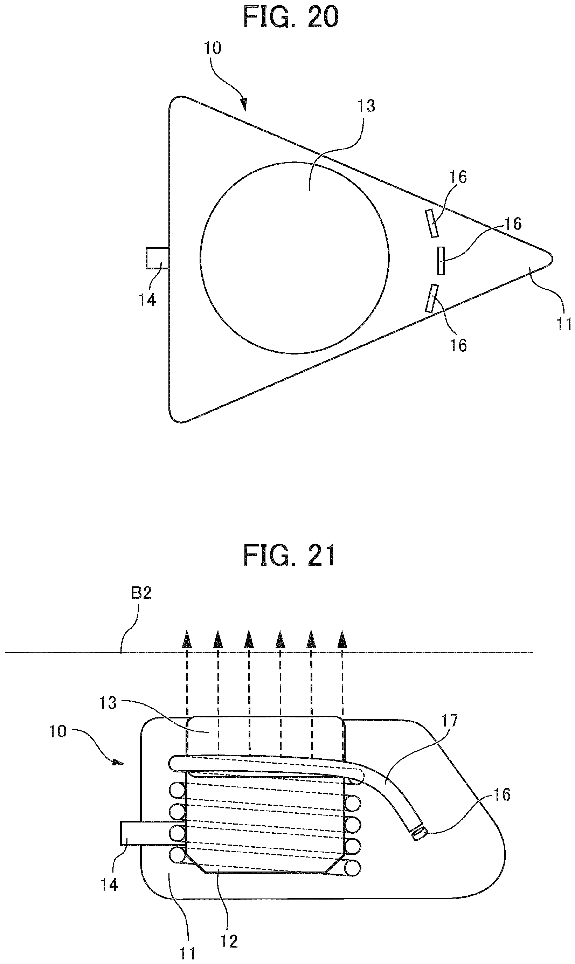

FIG. 19 is a diagram schematically showing a side cross-section of the medical instrument 10 according to the eleventh arrangement example. FIG. 20 is a diagram schematically showing a top surface of the medical instrument 10 according to the eleventh arrangement example. The container 12 is made entirely of resin. In the eleventh arrangement example, the position where the light emitters 16 are arranged is different from that in the first arrangement example. The light emitters 16 are disposed at any position within the main body 11 that does not contact the living body from the middle portion 12b toward a tip side 11a of the main body 11. The tip side 11a of the main body 11 refers to a side on which the injector 14 is not provided. Unlike the other locations, the tip side 11a has room to arrange the light emitter 16 at any position.

For example, as shown in FIG. 20, the respective light emitters 16 are arranged so that the center portions, i.e., the center axes, are oriented in a predetermined direction. The center portion (the center axis) refers to a portion where the light emitter 16 is most intensely illuminated. The predetermined direction refers to a direction passing through a predetermined location (e.g., the center) of the container 12 or a direction passing through a predetermined location (e.g., the center) of the soft portion 13. The number of the light emitters 16 is not limited to three.

In addition, it is considered that the light emitted from the respective light emitters 16 is scattered to the entire container 12 by providing the inner wall of the container 12 having an appropriate roughness.

According to such a configuration, the medical instrument 10 can irradiate all layers of the soft portion 13, all layers of the container 12, all layers of the skin, and the like with the light emitted from the light emitter 16, such that it is possible to reduce or prevent infection in all layers of the soft portion 13, all layers of the container 12, and all layers of the skin. Furthermore, according to the eleventh arrangement example, with the medical instrument 10, since the light emitted from the light emitter 16 passes through the skin B2, it is possible to grasp the position of the soft portion 13 from the outside, and assist the puncture operation of the injection needle 31 when the medicinal solution is injected into the container 12.

### (Twelfth Arrangement Example of Light Emitter)

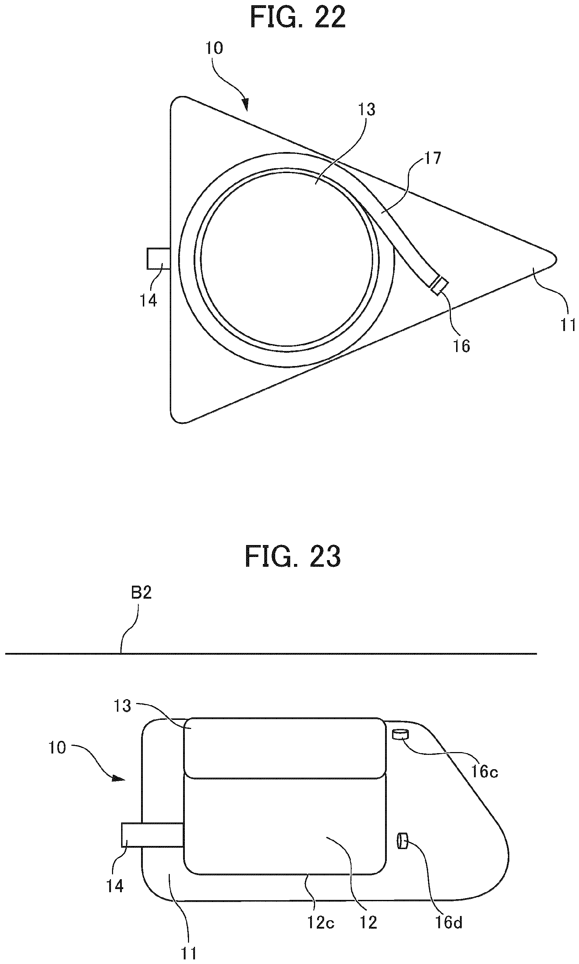

FIG. 21 is a diagram schematically showing a side cross-section of the medical instrument 10 according to the twelfth arrangement example. FIG. 22 is a diagram schematically showing a top surface of the medical instrument 10 according to the twelfth arrangement example. The container 12 is made entirely of resin.

The medical instrument 10 includes an optical fiber 17. The optical fiber 17 is free to bend into a variety of shapes, and is wound around the soft portion 13 and the container 12, as shown in FIG. 21. Furthermore, the optical fiber 17 has one end coupled to the light emitting surface of the light emitter 16. Here, the coupling method between the light emitter 16 and the optical fiber 17 is not particularly limited as long as it is a low loss coupling, and includes butt coupling, lens coupling, and other couplings. Furthermore, the optical fiber 17 is a material or structure that conveys light in the lateral direction and, for example, is a diffuse optical fiber.

According to such a configuration, since the medical instrument 10 causes the entire optical fiber 17 to emit light by using the light emitted from the light emitting section 16, all layers of the soft portion 13 and all layers of the container 12 can be irradiated by the light emitted from the optical fiber 17, thereby reducing or preventing infection in all layers of the soft portion 13 and all layers of the container 12. Furthermore, according to the twelfth arrangement example, with the medical instrument 10, since the light emitted from the side surface of the optical fiber 17 wound around the soft portion 13 passes through the skin B2, it is possible to grasp the position of the soft portion 13 from the outside, and assist the puncture operation of the injection needle 31 when the medicinal solution is injected into the container 12.

### (Thirteenth Arrangement Example of Light Emitter)

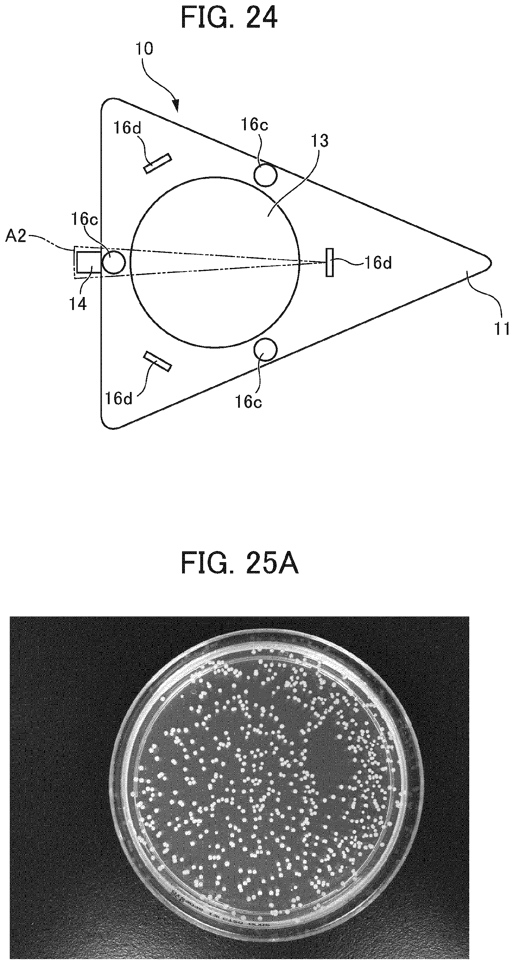

The medical instrument 10 may include a light emitter 16c that contributes to a marker function and a light emitter 16d that contributes to the reduction or prevention of an infection. Hereinafter, the thirteenth arrangement example of the light emitter will be described. FIG. 23 is a diagram schematically showing a side cross-section of the medical instrument 10 according to the thirteenth arrangement example. FIG. 24 is a diagram schematically showing a top surface of the medical instrument 10 according to the thirteenth arrangement example. The container 12 is made entirely of resin.

The light emitters 16c are disposed around the soft portion 13. More specifically, the light emitters 16c include three light emitters, and are arranged at intervals of about 120 degrees around the soft portion 13. The number of the light emitters 16c is not limited to three.

The light emitter 16c emits light in accordance with the proximity of the power transmitter 20 (the second coil 21). The light emitted from the light emitter 16c can be visually recognized from outside the body through the skin B2. The top surface of the soft portion 13 is located in the vicinity of the center of the locations where light is emitted at three locations. Furthermore, according to the thirteenth arrangement example, with the medical instrument 10, since the light emitted from the light emitter 16c passes through the skin B2, it is possible to grasp the position of the soft portion 13 from the outside, and assist the puncture operation of the injection needle 31 when the medicinal solution is injected into the container 12.

The light emitter 16d is disposed around the bottom portion 12c of the container 12. More specifically, the light emitter 16d includes three light emitters, which are arranged at intervals of about 120 degrees around the bottom portion 12c of the container 12. The number of the light emitters 16d is not limited to three. Since the light emitted from the light emitter 16d irradiates all layers of the soft portion 13, all layers of the container 12, all layers of the skin, and the like, it is possible to reduce or prevent infection in all layers of the soft portion 13, all layers of the container 12, and all layers of the skin.

Therefore, according to the thirteenth arrangement example, since the medical instrument 10 includes the light emitter 16c and the light emitter 16d, it is possible to exhibit a marker function when puncturing with the injection needle 31 to inject the medicinal solution into the container 12, and it is also possible to achieve the reduction or prevention of infection, in addition to hygiene when puncturing with the injection needle 31.

Furthermore, the treatment of injecting the medicinal solution into the container 12 is completed within about several minutes, but the irradiation of the light for reducing or preventing infection requires 5 minutes to 1 hour, depending on the center wavelength of the light emitted from the light emitter 16d and the magnitude of the irradiance. For example, the medical instrument 10 may receive power transmitted from the power transmitter 20, start light emission by the light emitter 16d to reduce or prevent infection, emit light by the light emitter 16c after a predetermined time has elapsed, specify the position of the soft portion 13, and inject a medicinal solution into the container 12. In addition, the medical instrument 10 may include a secondary battery therein. The medical instrument 10 charges the internal secondary battery with the power received from the power transmitter 20. The medical instrument 10 receives the power transmitted from the power transmitter 20, and emits light from the light emitters 16c and 16d, and specifies the position of the soft portion 13 by the light emitter 16c and punctures with the injection needle 31 while reducing or preventing infection by the light emitter 16d. The medical instrument 10 stops the power transmitted from the power transmitter 20 after the end of the puncture of the injection needle 31, and continues the light emission of the light emitter 16d by the power of the secondary battery to reduce or prevent infection. Furthermore, it may be configured to stop the power transmitted from the power transmitter 20 after charging the secondary battery. With such a configuration, the medical instrument 10 emits light from the light emitters 16c and 16d by the power of the secondary battery, specifies the position of the soft portion 13 by the light emitter 16c, and punctures with the injection needle 31 while reducing or preventing infection by the light emitter 16d. Therefore, since the medical instrument 10 can puncture with the injection needle 31 while causing the light emitter 16c to emit light by the power of the secondary battery, it is possible to puncture with the injection needle 31 without being limited to the location where the power transmitter 20 is disposed.

Furthermore, as shown in FIG. 24, it is preferable to provide one of the light emitters 16d at a position facing the injector 14 so as to irradiate the injector 14 with light. A2 in FIG. 24 indicates an irradiation site (range) A2 of the light (blue light) emitted from the light emitter 16d. In this way, the medical instrument 10 irradiates the entire injector 14 with light (blue light) emitted from the light emitter 16d, such that it is possible to reduce or prevent infection in the injector 14 which is an inlet of the catheter C.

### (Light Emitted from Light Emitter)

Hereinafter, light having a center wavelength of 600 nm to 1100 nm is referred to as red light. Furthermore, light having a center wavelength of 400 nm to 480 nm is referred to as blue light.

By irradiating the skin layer and the upper portion of the soft portion 13 with red light, the effect of the anti-inflammatory effect on these can be expected. This is because it is considered that irradiation with red light may have a direct effect on Staphylococcus aureus or may enhance the action of neutrophils.

Furthermore, by irradiating the interior of the soft portion 13, the container 12, and the injector 14 with red light, an antibacterial effect on these can be expected. This is because it is considered that irradiation with red light has a direct effect on Staphylococcus aureus.

Furthermore, by irradiating the interior of the soft portion 13, the container 12, and the injector 14 with blue light, a more positive antibacterial effect on these can be expected.

In addition, in the above description, irradiation of light from the light emitter 16 disposed in the medical instrument 10 has been described; however, the present invention is not limited to this configuration (a configuration in which light is irradiated from the interior of the living body B1), and irradiation of light may be performed from outside the living body B1.

### (Confirmation Test of Antibacterial Effect by Irradiation with Red Light)

Confirmation tests of antibacterial effect by the irradiation of red light were carried out by the following method. Two milliliters of phosphate-buffered saline adjusted to 1.5×10³/ml of methicillin-resistant Staphylococcus aureus (MRSA) was added to the agar-culture medium, and samples irradiated with red light from a planar light source using a red LED having a center wavelength of 680 nm at different radiant exposures were prepared (n=2). Then, each sample was cultured in an incubator set at 37°C for 48 hours, and the number of colonies formed on the agar-culture medium was counted. The radiant exposure of red light for each sample was set as 10 [J/cm²], 30 [J/cm²], and 60 [J/cm²]. In addition, the illumination intensity of red light was set as 12 [mW/cm²] for all the samples. As a control, a sample not irradiated with red light was used.

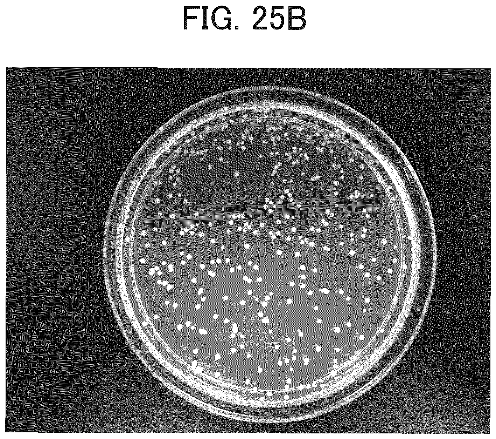

FIG. 25A is an image showing a status of methicillin-resistant Staphylococcus aureus (MRSA) colonies formed on a control agar-culture medium after 48 hours of incubation. FIG. 25B is an image showing the status of the colonies formed on an agar-culture medium after red-light irradiation at a radiant exposure of 60 [J/cm²] and after 48 hours of incubation. As shown in FGIS. 25A and 25B, colonies were formed on both the control and the sample irradiated with red light of 60 [J/cm²], and it was confirmed that the number of colonies in the sample irradiated with red light of 60 [J/ cm²] was smaller than the number of colonies in the control.

**[Table 1]**

| | RADIANT EXPOSURE (J/cm²) | | | |
|---|---|---|---|---|
| | CONTROL | 10 | 30 | 60 |
| NUMBER OF COLONIES (WHEN NORMALIZED BY NUMBER OF COLONIES OF CONTROL) | 1 | 0.97 | 0.82 | 0.5 |

Table 1 shows the average number of colonies (n=2) according to each irradiation condition normalized by the number of colonies in the control. As shown in Table 1, the number of colonies decreases as the radiant exposure of red light increases. Furthermore, the temperature increase during irradiation with red light is 2°C or less, and thus, it is considered that there is no effect on antibacterial action by temperature. These results strongly suggest the antibacterial effect of irradiation with red light. In addition, the antibacterial effect (Photobiomodulation) on cells/organisms due to weak light is generally enhanced by the presence of neutrophils, which are blood components; however, in this experimental system, the antibacterial effect is exhibited even in the absence of neutrophils. Therefore, this test result strongly suggests that the antibacterial effect can be obtained even in the living tissue in which the blood components exist in the surrounding tissue, and that the infection related to the medical instrument implanted in a body can be reduced or prevented by the weak light. In addition, in a living body in which neutrophils are present, an antimicrobial effect can be expected with a smaller radiant exposure.

While some of the embodiments of the present application have been described in detail based on the drawings, these are illustrative, and it is possible to implement the present invention in other forms which have been subjected to various modifications and improvements based on the knowledge of those skilled in the art, including the aspects described in the disclosure of the present invention. In particular, the position where the light emitters 16 are arranged, the number of the light emitters 16, the irradiation times and periods of the light emitters 16, the center wavelengths of the light emitters 16, and other conditions described above are examples, and are not limited thereto.

### EXPLANATION OF REFERENCE NUMERALS

1 medical device
10 medical instrument
11 main body
11a tip side
12a opening
12b middle portion
12c bottom portion
12d hole
13 soft portion (septum)
14 injector (catheter port)
15 power receiver
15a first coil
15b power receiving circuit
16, 16_1, 16_2 light emitter
16a first light emitter
16b second light emitter
17 optical fiber
20 power transmitter
31 injection needle

## Claims

1. An implantable medical instrument for use within a body, the medical instrument including a container that has an opening and holds a medicinal solution, and a soft portion that closes the opening, the medical instrument comprising:
a power receiver that receives power transmitted externally; and
a light emitter that emits light by way of the power received by the power receiver,
wherein the light emitter includes at least one of a first light emitter that emits light having a center wavelength of 600 nm or more and 1100 nm or less, and a second light emitter that emits light having a center wavelength of 400 nm or more and 480 nm or less.

2. The medical instrument according to claim 1, wherein the light emitter is provided around the soft portion, and irradiates at least a top surface of the soft portion.

3. The medical instrument according to claim 1, wherein the light emitter is disposed around a middle portion or a bottom portion of the container, and irradiates at least an interior of the container and the soft portion.

4. The medical instrument according to any one of claims 1 to 3, wherein the light emitter includes at least the first light emitter, and
wherein a radiant exposure of the light emitted from the first light emitter is in a range from 1.0 to 100.0 J/cm² at an irradiation target site.

5. The medical instrument according to any one of claims 1 to 4, wherein the light emitter includes at least the first light emitter, and
wherein the first light emitter emits light having a center wavelength of 620 nm, 660 nm, 680 nm, 760 nm, or 820 nm.

6. The medical instrument according to any one of claims 1 to 3, wherein the light emitter includes at least the second light emitter, and
wherein a radiant exposure of the light emitted from the second light emitter is in a range from 32 to 125 J/cm² at an irradiation target site.

7. A medical device comprising:
a medical instrument according to any one of claims 1 to 6; and
a power transmitter that transmits predetermined power,
wherein the medical instrument further includes a power receiver that receives power transmitted from the power transmitter.
